# EUROPEAN PATENT APPLICATION

(11) **EP 4 116 991 A1**
(43) Date of publication of application: **11.01.2023**
(21) Application number: 21461568.4
(22) Date of filing: 10.07.2021
(51) Int. Cl.: G16H 50/20

(54) **A SYSTEM AND A METHOD FOR PREDICTING TIME OF OVULATION**

(71) Applicant: Edge One Solutions Sp. z o.o., 02-676 Warszawa (PL)
(72) Inventor: CZERNECKI, Lukasz, Laka (PL); LOBEJKO, Mariusz, Lublin (PL)
(74) Representative: Kancelaria Eupatent.pl Sp. z.o.o

(57) **Abstract**

A computer-implemented method for predicting time of ovulation of a female, the method comprising: reading (302) historical measurement data of the female, the measurement data including galvanic skin response (GSR) measurements; reading (303) clinical measurement data of other females, the clinical measurement data including GSR measurements; training (304) an artificial intelligence prediction module (132) by the historical measurement data of the female related to at least one previous menstrual cycle and clinical measurement data of other females related to menstrual cycles; and providing (305) to the trained artificial intelligence prediction module (132) recent historical measurement data of the female to receive a predicted time of ovulation.

## Description

### TECHNICAL FIELD

The present invention relates to predicting time of ovulation, in particular based on measurements of galvanic skin response.

### BACKGROUND

There are numerous known methods related to predicting time of ovulation. The methods involve analysis of various physiological parameters of body, such as temperature, heart rate, breathing rate, perfusion, vascular activity or galvanic skin response.

In view of a constant need for more accurate predictions, there is a need to provide an alternative system for predicting time of ovulation.

### SUMMARY

The object of the invention is a computer-implemented method for predicting time of ovulation of a female, the method comprising: reading historical measurement data of the female, the measurement data including galvanic skin response (GSR) measurements; reading clinical measurement data of other females, the clinical measurement data including GSR measurements; training an artificial intelligence prediction module by the historical measurement data of the female related to at least one previous menstrual cycle and clinical measurement data of other females related to menstrual cycles; and providing to the trained artificial intelligence prediction module recent historical measurement data of the female to receive a predicted time of ovulation.

The historical measurement data may include GSR measurement parameters related to time segments, wherein the parameters include at least one of: a rounding value, a mod value, an average value, a minimum value, a maximum value of measurement values measured during that time segment.

The GSR parameters may be generated by pre-processing body measurement data collected over a plurality of time segments and comprising GSR measurements, temperature measurements and inter beat interval (IBI) measurements, wherein the GSR measurement parameters are stored for these time segments, for which IBI measurements were higher than a threshold and for which temperature measurements were lower than a threshold, wherein for other time segments a default parameter value is stored.

The clinical measurement data may include measurement data of other females sharing at least one of: age, illness, living conditions.

Preferably, the recent historical measurement data used for the step of predicting does not include at least part of the historical measurement data used for the step of training.

The method may comprise use of LGBMRegressor and XGBRegressor algorithms for training the AI prediction module.

The method may comprise use of XGBOOST, LGBM, ANN algorithms for prediction using the AI prediction module.

The method may comprise calculating an average of the output of XGBOOST, LGBM, ANN algorithm predictions.

The object of the invention is also a system for predicting time of ovulation of a female, the system comprising a prediction module comprising: a data storage configured to store historical measurement data of the female, and clinical measurement data of other females; the measurement data including galvanic skin response (GSR) measurements; an artificial intelligence (AI) prediction module controller module which is a neural network comprising training algorithms and prediction algorithms; and a controller module configured to: read from the data storage historical measurement data of the female, the measurement data including galvanic skin response (GSR); read from the data storage clinical measurement data of other females, the clinical measurement data including galvanic skin response (GSR); train the AI prediction module by the historical measurement data of the female related to at least one previous menstrual cycle and clinical measurement data of other females related to menstrual cycles; and provide to the trained AI prediction module recent historical measurement data of the female to receive a predicted time of ovulation.

The prediction module may be configured to communicate with communication modules that receive data from a data source in form of a wrist band comprising a GSR sensor, a temperature sensor and an IBI sensor.

By taking into account (during training and prediction phase of the AI prediction network) only GSR parameters calculated for time segments for which the user had high IBI values (indicating that the user is resting) and low temperature values (disregarding periods of illness with high temperature), the presented method can output a prediction of time of ovulation based on little amount of input data (only GSR measurements) which are of high value (collected for a healthy user during periods of rest). Thereby, the method can provide effective and reliable predictions.

These and other features, aspects and advantages of the invention will become better understood with reference to the following drawings, descriptions and claims.

### BRIEF DESCRIPTION OF DRAWINGS

Various embodiments are herein described, by way of example only, with reference to the accompanying drawings, wherein:
Fig. 1 shows an example functional schematic of the computer-implemented system for predicting time of ovulation;
Fig. 2 shows a method for data pre-processing;
Fig. 3 shows a procedure for predicting the time of ovulation;
Fig. 4 shows a structure of the AI prediction module;
Fig. 5 shows the structure of a computer system for implementing the functionality of the modules of Fig. 1.

### DETAILED DESCRIPTION

The following detailed description is of the best currently contemplated modes of carrying out the invention. The description is not to be taken in a limiting sense, but is made merely for the purpose of illustrating the general principles of the invention.

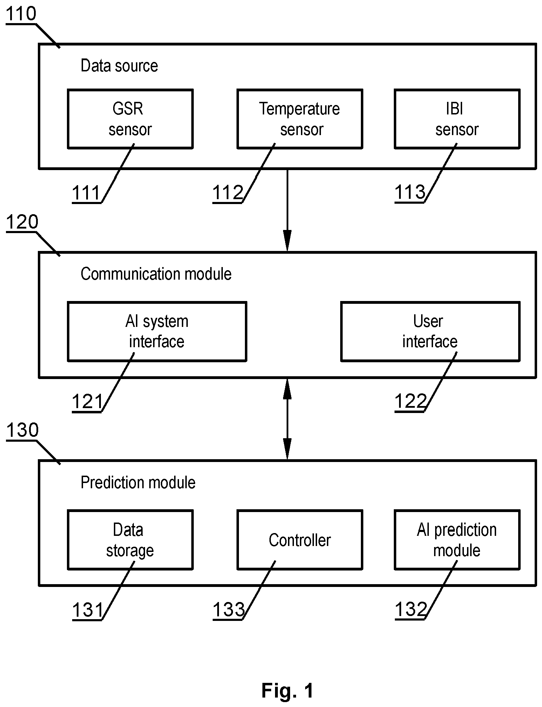

Fig. 1 shows an example functional schematic of the computer-implemented system for predicting time of ovulation. The system comprises three main components: a data source 110, a communication module 120 and a prediction module 130.

The data source 110 can have a form of a wrist band with sensors mounted thereon, such as a Galvanic Skin Response (GSR) sensor 111 (preferably, a skin contact sensor), a temperature sensor 112 (preferably, a contactless sensor) and an Inter Beat Interval (IBI) sensor 113 (preferably, a contactless sensor). The sensors are configured to measure corresponding parameters with a predefined frequency, for example the GSR sensor 111 and the temperature sensor 112 may collect data with a frequency of a few Hertz (e.g. 4 Hz, namely 4 measurements per second) and the IBI sensor may provide inter beat interval in a rate according to the heart beat.

The communication module 120 is configured to read the measurement data, including the GSR measurements, temperature measurements and IBI measurements, for example from the data source 110 in the form of the wrist band, but also from other kinds of data sources (e.g. from a data interface configured to transmit data from other types of measurement devices or to read data from medical systems or other ovulation prediction systems). The measurement data can be read via a known interface, wired or wireless, such as WiFi or Bluetooth. Data can be read in real time, periodically or on demand (upon push from the data source or pull initiated by the communication module). The communication module 120 is further configured to communicate with the prediction module 130 for transmitting the measurement data to the analysis module 130. The prediction module 130 can be communicated via a known interface, wired or wireless, such as WiFi, GSM or Ethernet. The measurement data is sent to the prediction module in real time, periodically or on demand (upon push from the communication module or pull initiated by the prediction module). The measurement data read from the data source 110 is stored in a local memory of the communication module 120 until it is transmitted to the prediction module 130, and upon confirmation of a safe receipt, it can be deleted from the memory of the communication module 120. The communication module 120 further comprises a user interface for configuring the system, inputting additional data (such as user's parameters such as age, weight, last ovulation day) and outputting prediction results - for example, via a touch screen display.

The communication module 120 can be integrated with the data source 110, e.g. may have a form of a smartwatch with integrated user interface and communication modules. Alternatively, the communication module 120 can be implemented as a software installed in a user device such as a smartphone or a personal computer, which communicates with the wrist band functioning solely as the data source 110.

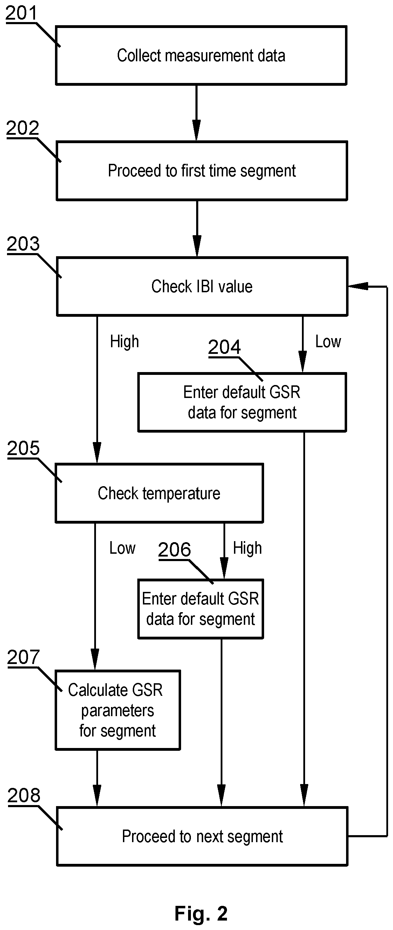

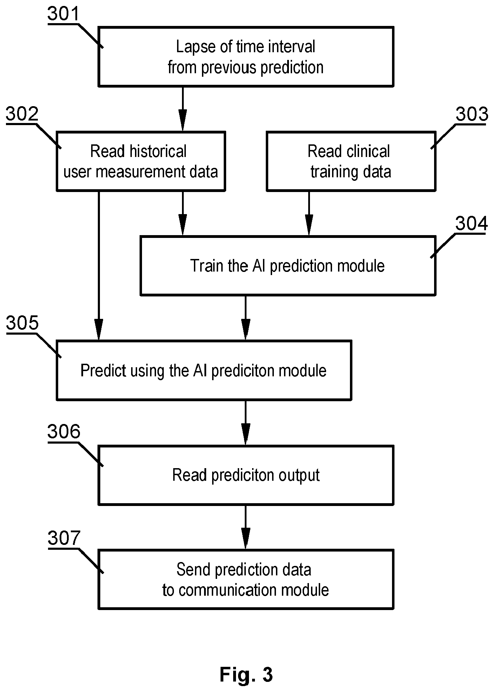

The prediction module 130 can be implemented as a server accessible in a network. For example, it can be implemented in one or more physical or virtual servers available in cloud with access to data storage and operating software implementing the artificial intelligence module functionality. The data storage 131 is configured to store measurement data of the users of the system received from their communication modules 120. The artificial intelligence module 132 is configured to perform prediction of ovulation time based on the user data. A controller 133 is used to control the operation of the system in accordance with the procedures of Figs. 2 and 3.

The data collected from the data source has a form of a series of measurements (such as 4 readings of GSR value per second, 4 readings of temperature value per second and consecutive IBI times). In order to optimize data for further processing, data are divided into segments having a specific length, such as 1 hour. The preprocessing can be performed at the communication module 120 (to limit the amount of data transmitted to the prediction module) or at the prediction module 130 (such as to limit the workload a the communication module).

Fig. 2 shows an algorithm of the pre-preprocessing. First, the measurement data are collected in step 201 (i.e. read from the data storage if pre-stored or pulled from the data source 110 via the communication module 120). Next, in step 202 the procedure reads data from the first time segment (e.g. the first hour). In step 203 it is checked what is the average IBI value for that segment. If the IBI value is high (e.g. higher than an average value for the whole day) it indicates that during this time segment the user was active, e.g. moving around, and the GSR readings may be imprecise. In that case, in step 204 default GSR data is stored for that segment - such as a zero value or the value of the preceding time segment. If the IBI value is low (e.g. lower than the average value for the whole day) it indicates that during this time segment the user was inactive (e.g. resting or sleeping) and the GSR readings are supposed to be precise. In that case, in step 205 it is checked what was the average temperature during that time segment. If the temperature was high (e.g. higher than 37 degrees C) it may indicate that the user is not healthy and the GSR readings may be misleading, in that case in step 206 default GSR data are entered for that time segment (e.g. a zero value or the value of the preceding time segment). If the temperature was low (or normal, such as lower than 37 degrees C) it indicates that the user is healthy and the GSR readings are useful - in that case, in step 207 the GSR statistical parameters are extracted for that segment, such as: a rounding of values, an mod, an average, a minimum value, a maximum value. One or more of such statistical parameters are then stored for that segment in the data storage 131. Therefore, the data storage can include for each user historical measurement data related to past measurements, stored in a form of records, each record related to a single time segment and indicating:
- user ID;
- measurement day;
- measurement time segment (from 1 to 24, if segments are of 1 hour);
- GSR measurement parameters (e.g. an average, min and max value measured within that segment);
- indication that ovulation occurred at the particular segment.

Other similar data structures can be used as well.

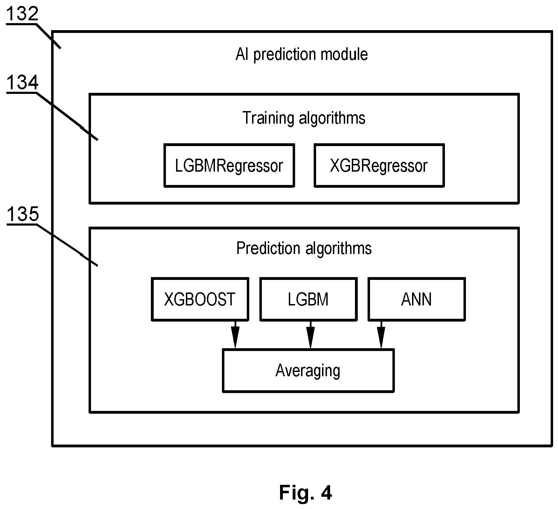

Fig. 3 shows a procedure for predicting the time ovulation. The procedure is performed individually for each user in a periodic manner, in specific time intervals, for example every 8 hours. Therefore, it starts in step 301 upon lapse of a time interval since previous recommendation. Then, user historical measurement data are read in step 302 from the data storage 131 that include the past data from a specific time period, for example from the last 12 months (or less if less data is available) or even all available past data for that user. In one embodiment, the system may initiate this prediction procedure only if a predetermined amount of data is available, for example data at least from the past two menstrual cycles. If less data is available, the system may be configured to provide prediction based on mathematical algorithms or predefined tables, without the use of the artificial intelligence module, due to lack of data. Next, the clinical training data are read in step 303 that include past measurement data of other females that can be helpful to predict the outcome for the current user. For example, the clinical training data can be selected from the larger set such as to correspond to other females selected according to similarity to the current user (e.g. having similar age, medical record (e.g. illnesses), life environment etc). The clinical training data comprise measurement data from past menstrual cycles of the other subjects. The user data and clinical data are then input in step 304 to the AI prediction module 132 set to a training mode and to be processed by the training algorithms as explained in Fig. 4. For the training phase, all user historical measurement data can be used or only part of historical measurement data (e.g. excluding the current menstrual cycle and/or one or more previous cycles). Next, when the AI prediction module 132 is trained, in step 305 the recent user data, i.e. all available historical measurement data or data from only the current menstrual cycle and/or one or more previous cycles, is input to the AI prediction module 132 set to a prediction mode and to be processed by the prediction algorithms as explained in Fig. 4. The prediction result is read in step 306 and sent to the communication module 120 in step 307 to be presented to the user.

Fig. 4 shows a structure of the AI prediction module 132. In comprises a set of training algorithms 134 and a set of prediction algorithms 135.

The AI prediction module 132 can be implemented as an artificial neural network (ANN). For example, the network may having three hidden fully connected layers with activation functions (relu, relu, linear) and an output layer with an activation function (sigmoid). After each hidden layer there can be 10% of randomly scattered neurons. The hidden layers may be devoid of regularization functions L1, L2.

For training, a LGBMRegressor and XGBRegressor algorithms can be used. The LGBMRegressor algorithm can be configured as follows:
- the loss function and the monitoring function: MAE (mean absolute error);
- the training is terminated if the validating monitoring function does not improve for 10 consecutive iterations;
- number of random trees: 10000;
- no limitation as to the size of trees;
- no limitation as to the number of leaves at the ends of branches.
The XGBRegressor algorithm can be configured as follows:
- the loss function: MSE (mean square error);
- the monitoring function: MAE (mean absolute error);
- the training is terminated if the validating monitoring function does not improve for 10 consecutive iterations;
- number of random trees: 10000;
- no limitation as to the size of trees;
- no limitation as to the number of leaves at the ends of branches.
For prediction, the following algorithms can be used: XGBOOST, LGBM, ANN. Each prediction algorithm provides a prediction output and an average (with equal or different weights) of these outputs is provided as the predicted time of ovulation. In addition, the currently predicted value can be averaged with one or more (e.g. from within the past 24 hours) predicted prediction values.

The prediction value may have a form of e.g. a value specifying the number of hours left till the expected time of ovulation. When presented to the user, it can be added to the current time and date such as to present the time and date of the expected ovulation.

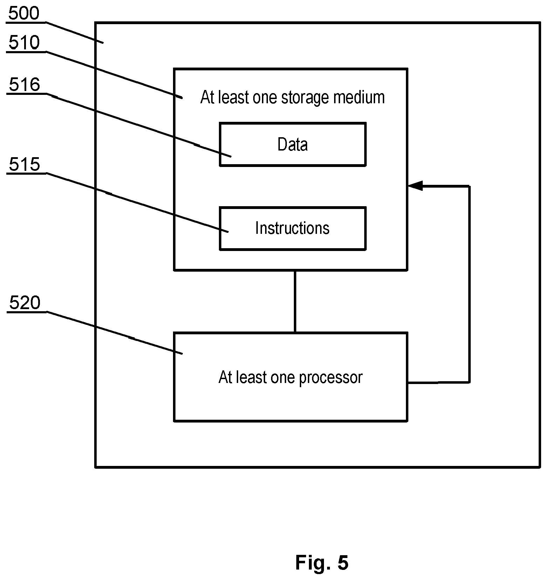

The functionality described herein can be implemented in a computer system 400, such as shown in Fig. 5. The system 500 may include at least one nontransitory processor-readable storage medium 510 that stores at least one of processor-executable instructions 515 or data 516; and at least one processor 520 communicably coupled to the at least one nontransitory processor-readable storage medium 510. The at least one processor 520 may be configured to (by executing the instructions 515) provide the functionality of the communication module 120 or the prediction module 130, in particular of the controller module 133 or the AI prediction module 132.

While the invention has been described with respect to a limited number of embodiments, it will be appreciated that many variations, modifications and other applications of the invention may be made. Therefore, the claimed invention as recited in the claims that follow is not limited to the embodiments described herein.

## Claims

1. A computer-implemented method for predicting time of ovulation of a female, the method comprising:
- reading (302) historical measurement data of the female, the measurement data including galvanic skin response (GSR) measurements;
- reading (303) clinical measurement data of other females, the clinical measurement data including GSR measurements;
- training (304) an artificial intelligence prediction module (132) by the historical measurement data of the female related to at least one previous menstrual cycle and clinical measurement data of other females related to menstrual cycles; and
- providing (305) to the trained artificial intelligence prediction module (132) recent historical measurement data of the female to receive a predicted time of ovulation.

2. The method according to claim 1, wherein the historical measurement data include GSR measurement parameters related to time segments, wherein the parameters include at least one of: a rounding value, a mod value, an average value, a minimum value, a maximum value of measurement values measured during that time segment.

3. The method according to claim 2, wherein the GSR parameters are generated by pre-processing body measurement data collected over a plurality of time segments and comprising GSR measurements, temperature measurements and inter beat interval (IBI) measurements, wherein the GSR measurement parameters are stored for these time segments, for which IBI measurements were higher than a threshold and for which temperature measurements were lower than a threshold, wherein for other time segments a default parameter value is stored.

4. The method according to any of previous claims, wherein clinical measurement data include measurement data of other females sharing at least one of: age, illness, living conditions.

5. The method according to any of previous claims, wherein the recent historical measurement data used for the step of predicting (305) does not include at least part of the historical measurement data used for the step of training (304).

6. The method according to any of previous claims, comprising use of LGBMRegressor and XGBRegressor algorithms for training (304) the AI prediction module (132).

7. The method according to any of previous claims, comprising use of XGBOOST, LGBM, ANN algorithms for prediction (305) using the AI prediction module (132).

8. The method according to claim 7, comprising calculating an average of the output of XGBOOST, LGBM, ANN algorithm predictions.

9. A system for predicting time of ovulation of a female, the system comprising a prediction module (130) comprising:
- a data storage (131) configured to store historical measurement data of the female, and clinical measurement data of other females; the measurement data including galvanic skin response (GSR) measurements;
- an artificial intelligence (AI) prediction module controller module (132) which is a neural network comprising training algorithms (134) and prediction algorithms (135); and
- a controller module (133) configured to:
- read (302) from the data storage (131) historical measurement data of the female, the measurement data including galvanic skin response (GSR);
- read (303) from the data storage (131) clinical measurement data of other females, the clinical measurement data including galvanic skin response (GSR);
- train (304) the AI prediction module (132) by the historical measurement data of the female related to at least one previous menstrual cycle and clinical measurement data of other females related to menstrual cycles; and
- provide (305) to the trained AI prediction module (132) recent historical measurement data of the female to receive a predicted time of ovulation.

10. The system according to claim 9, wherein the prediction module (130) is configured to communicate with communication modules (120) that receive data from a data source (110) in form of a wrist band comprising a GSR sensor (111), a temperature sensor (112) and an IBI sensor (113).
